# EUROPEAN PATENT SPECIFICATION

(11) **EP 3 972 486 B1**
(45) Date of publication and mention of the grant of the patent: **31.01.2024**
(21) Application number: 20734795.6
(22) Date of filing: 22.05.2020
(51) Int. Cl.: A61B 5/00, G16H 20/10, G16H 40/63

(54) **WEARABLE DEVICE FOR RECORDING HEADACHE INTENSITY AND MEDICATION**
TRAGBARE VORRICHTUNG ZUR ERFASSUNG VON KOPFSCHMERZINTENSITÄT UND MEDIKATION
DISPOSITIF POUVANT ÊTRE PORTÉ POUR ENREGISTRER L'INTENSITÉ DE CÉPHALÉE ET UNE MÉDICATION

(30) Priority: 22.05.2019 GB 201907237
(43) Date of publication of application: 30.03.2022
(73) Proprietor: Ceftronics Limited, Gloucester Gloucestershire GL3 4SB (GB)
(72) Inventor: WAKERLEY, Benjamin, Gloucester Gloucestershire GL3 4SB (GB)
(74) Representative: Games, Robert Harland
(86) International application number: PCT/GB2020/051249
(87) International publication number: WO 2020/234604

(56) References cited:
- WO-A1-00/47108
- WO-A1-2009/144325
- KR-A- 20170 089 727
- US-A1- 2008 058 664
- US-A1- 2011 275 907

## Description

The present invention relates to a wearable device for recording headache intensity and medication, particularly but not exclusively where the headache is a migraine or suspected migraine.

### BACKGROUND TO THE INVENTION

Migraine is a common cause of headache and affects around 15% of the global population. According to the 2015 Global Burden of Disease Study, migraine ranks as the third most common cause of disability in the under 50s. Chronic migraine (≥15 days of headache per month, of which >8 are migrainous, for ≥3 months) affects 2% of the population. It is estimated that the UK population loses 25 million days from work or school because of migraine.

Headache diaries are very useful in the management of migraine and provide important information to patients and treating physicians. They provide information on the frequency and severity of headaches and also on the use of headache medications. Simple paper diaries have proved most effective, but sometimes become lost or damaged, and so are unreliable.

Headache recordal apps for mobile phones are also available, for iOS and Android devices, but they are often overcomplicated and don't necessarily provide the information required to monitor and manage migraine.

A greater problem is that migraine is highly debilitating, affecting vision to the extent that a person may not be able to see, the ability to think to the extent that a simple task becomes impossible, and a feeling of nausea. In some cases, injector pens are used to medicate with pain relief, but the symptoms can be so severe that some people cannot successfully assemble the pen to make the injection.

A screen on a mobile device tends to be bright, with the text backlit and small in size, and so during a migraine, it is not generally possible to use the device. Consequently, the use of an App becomes impossible during a migraine and so the migraine severity and use of painkillers cannot be noted at the time. Therefore, this information has to be recorded at a later time, i.e. when the migraine has passed, and often the information is not recorded accurately, or even at all, particularly if migraines are occurring with regular frequency.

Individuals who frequently use painkillers are at an increased risk of developing medication-overuse headache (MOH). This is a concern for individuals suffering for migraine and/or tension-type headaches who use painkillers. The likelihood of developing MOH relates to i) the type of medication used and ii) the frequency of use. For example, individuals who use opiate-based medication on more than 10 days in a given month for > 3 months are likely to develop MOH.

WO 00/47108 discloses an ambulatory monitor comprising: a fastener for attaching said monitor to an ambulatory person to be monitored; a display; an input interface; and a query generator which generates queries to said display and which receives responses to said queries using said input interface. A method of detecting a changing in pain level is also disclosed and comprises tracking movements of at least a portion of a person; and analysing said tracked movements to identify changes in movement caused by a change in pain level. A method of data sensing is also disclosed and comprises automatically logging data of an ambulatory patient; analysing said data to determine at least one aspect of non-suitability of said logged data; automatically querying said patient to provide data which improves said at least one aspect of non-suitability. Also disclosed is a method of entering multi-state information which comprises displaying, on a donned device, a scale of values, including an indication of a particular value; entering, using said device, a value, which entered value is indicated using said indication; and storing said enter value, in said device, for later analysis. A monitor network is further disclosed comprising a first monitor, worn by a first person, which first monitor generates an alert to said first person; and a second monitor, worn by a second person, synchronised with said first monitor, which generates an alert to said second person responsive to said first monitor.

It is an object of the present invention to provide a wearable device which reduces or substantially obviates the aforementioned problems.

### STATEMENT OF INVENTION

According to the present invention, there is provided a wearable device as claimed in claim 1.

Optional features are claimed in claims 2 to 15.

In use, the first input button is pressed to record a medicament dosing event. To record headache severity level, the user presses the second button a number of times within the predetermined time period. For example, three presses within ten second might record a "level 3" headache on a scale of 1 to 3, 3 being most severe. Once the second button has not been pressed for a few seconds (the first delay) the vibration motor is activated to feedback the value entered, for example, three short vibrations to indicate that a "level 3" headache has been recorded.

In an embodiment where there are a relatively small number of headache severity levels, for example 3, if the second input button is pressed repeatedly the count may roll over and scroll through the various levels. After the number of presses for the maximum severity level, in some embodiments the count may roll to zero (i.e. no headache) or one (i.e. the least severe headache). For example, where three is the most severe headache level, four presses of the second input button may record either no headache (zero) or a mild headache (level one). After a delay following the final press of the second input button, the vibration motor vibrates the appropriate number of times to indicate the level being recorded. If incorrect, embodiments may provide an opportunity for the user to cancel the input and start again, as described in more detail below.

Advantageously, a person wearing the device is able to record a headache at the time that it occurs and also record the taking of a painkiller, without reference to a screen. Separate buttons enable straightforward operation, when debilitated by a headache, and the feedback vibrations corresponding to the inputs enable a person to confirm that the correct inputs have been made. Having the operation of the second input button correspond to a predetermined scale representing the headache level provides a simple method to record the severity of the headache.

The wearable device may include a display for displaying information to a user. The screen may be disposed on the front face of the body. The displayed information may be based on the data stored in the memory. The displayed information may also be modified to indicate the number of events within a given period.

The display allows the user to validate input either after or during the headache, preferably after the headache.

The wearable device may include a communication means for transmitting the recorded data to a remote computer.

The communication means may include a wired communication means. The communication means may include a wireless communication means.

A plurality of vibration patterns may be provided. Each vibration pattern has at least one vibration pulse. Each vibration pattern may last for a period of time. The vibration patterns may uniquely identify operation of an input button and/or the successful recording or removal of an event or headache level.

The wearable device may include a buzzer. The buzzer may produce auditory feedback based on the operation of the input button and/or the successful recording or removal of an event or headache level. The buzzer may sound in concert with the vibration motor.

The auditory feedback of the buzzer may uniquely identify operation of any input button and/or successful recording or removal of an event or headache level.

By providing different feedback periods and/or patterns for each input button it is possible for a user, when debilitated by a headache, to confirm that the correct inputs have been made and/or information has been recorded/deleted.

There may be a delay between operation of the first button and the medicament dosing event being permanently recorded in the memory. The vibration motor may be controlled to operate within the delay to indicate the medicament dosing event based on the operation of the first input button. The vibration motor may be controlled to represent the medicament dosing event has been registered prior to being recorded in the memory.

Operation of the first input button for at least a first predetermined length of time may record the medicament event. For example, the first input button may be depressed and held in that position for the first predetermined length of time. The vibration motor may vibrate for a period corresponding to the first predetermined length of time.

Subsequent operation of the first input button for at least a second predetermined length of time within a first predetermined period after the first predetermined length of time may remove the medicament event, preventing it from being permanently recorded in the memory. For example, the first input button may be depressed and held in that position for the second predetermined length of time. The vibration motor may vibrate for a period corresponding to the second predetermined length of time.

Requiring the first input button to be operated for a predetermined length of time ensures that a medicament event cannot be accidently recorded or removed.

Providing the vibrational feedback prior to the medicament dosing event being permanently stored in the memory allows a user debilitated by the headache to determine or validate their input without reference to a screen, and cancel an erroneous input.

The headache severity level is be stored in the memory after the predetermined time period.

Providing the vibrational feedback prior to the headache severity level being stored in the memory allows a user debilitated by the headache to determine or validate their input without reference to a screen, and cancel an erroneous input.

An initial operation of the second input button may initiate an input mode. The vibration motor may vibrate to indicate the input mode. During input mode, subsequent operation of the second input button within a predetermined period sets the headache level on a predetermined scale. The vibration motor may vibrate to indicate the headache level after a delay.

Requiring an initial operation of the second input button prior to setting the headache level ensures no headache level is recorded by mistake when the user is not suffering a headache.

The wearable device may further comprise a third input button for controlling the display.

A date and/or time may be recorded with the medicament event. A date and/or time may be recorded with the headache level.

The inclusion of time and/or date data allows for a user or medical professional to easily monitor trends and/or make referrals.

The wearable device may comprise a body. The body may house components, such as a memory, a vibration motor, a processor. The housing may have a front face, a rear face, side faces, a first end and a second end.

The first input button may be disposed on the front face. The first input button may be raised above the front face. Alternatively, the first input button may be flush with the front face.

The second input button may be disposed on one of the side faces. The second input button may be raised from the side face on which it is disposed. Alternatively, the second input button may be flush with the side face on which it is disposed.

The third input button may be disposed on the side face opposite the second input button. The third input button may be raised from the side face on which it is disposed. Alternatively, the third input button may be flush with the side face on which it is disposed.

The separated positioning of input buttons between the front and side of the device enables straightforward operation, when debilitated by a headache, and the feedback vibrations corresponding to the inputs enable a person to confirm that the correct inputs have been made. Furthermore, the separated position of the input buttons allows a user to identify the relevant input button without looking at the device or even opening their eyes, if significantly debilitated by a migraine.

Each input button may include a textured surface or a touch identification mark which assists a user to uniquely identify the button. The shape of each input button may be different to help a user differentiate between the buttons. Primarily, it is the placement of the buttons on separate surfaces which supports user differentiation of the buttons whilst suffering a migraine.

The wearable device may comprise a strap attached to the first and second ends of the body by means of a releasable fastener. The releasable fastener may also allow the strap to pivot about the ends of the body.

The strap may comprise a means for attaching two parts of the strap together so as to secure the wearable device around the user.

The wearable device may include a charging means for recharging a battery.

A plurality of conductors may be disposed on the side or rear of the body. At least some of the plurality of conductors may be used for charging. At least some of the plurality of conductors (whether or not used for charging) may be used for communication and transfer data. The plurality of conductors may correspond and interface, in use, with a plurality of conductors disposed on a dock, clamp or cable.

The wearable device may include a pedometer.

In a further aspect of the present invention there is provided a remote computer for storing and analysing medicament event data and headache level data, the remote computer comprises a display, a memory, a communication module and a processor, the processor configured to: communicate, through the communication modules, with a wearable device according to another aspect of the present invention; receive, through the communication module, data collected from the wearable device; store, in the memory, the received data; and analyse the received data.

The remote computer may generate an alert based on the analysed data.

The alert may be based on the number of medicament events meeting or exceeding a predetermined threshold.

The alert may be based on the number of headache events meeting or exceeding a predetermined threshold.

The remote computer may generate reports based on the received and/or analysed data.

The wearable device may be considered to be a wearable electronic headache diary or a wearable migraine recordal device.

The data recorded by the wearable device may be hidden from the user of the wearable device. The data may be provided to a medical professional for monitoring patients.

The combination of wearable device and remote computer can be considered as a system for recording and monitoring headache severity level and medicament dosing events.

Advantageously, communication and transmittal of data to a remote device allows medical professionals to easily monitor a patient's condition and make decisions on more reliable data. Producing alerts allows the user of the remote device to be aware of possible issues. For example, the alert may inform the user that the number of painkillers taken in a month has exceeded guidelines and a referral to a specialist should be made or no more painkillers should be taken. Furthermore, the user of the wearable device may self-monitor and visualise the data when the data is transfer to an application on a smart phone or other personal device such as a tablet.

### BRIEF DESCRIPTION OF THE DRAWINGS

For a better understanding of the present invention, and to show more clearly how it may be carried into effect, reference will now be made by way of example only to the accompanying drawings, in which:
Figure 1 shows a schematic view of a wearable device according to an aspect of the present invention;
Figure 2 shows a schematic isometric view of a wearable device according to any aspect of the present invention;
Figure 3 shows a schematic representation of the wearable device according to an aspect of the present invention;
Figures 4a and 4b shows an example of a screen displayed on a wearable device;
Figures 5a, 5b, 5c and 5d, shows an example of a screen displayed on a wearable device; and
Figure 6 shows a schematic representation of wearable device communicating with a remote computer.

### DESCRIPTION OF PREFERRED EMBODIMENTS

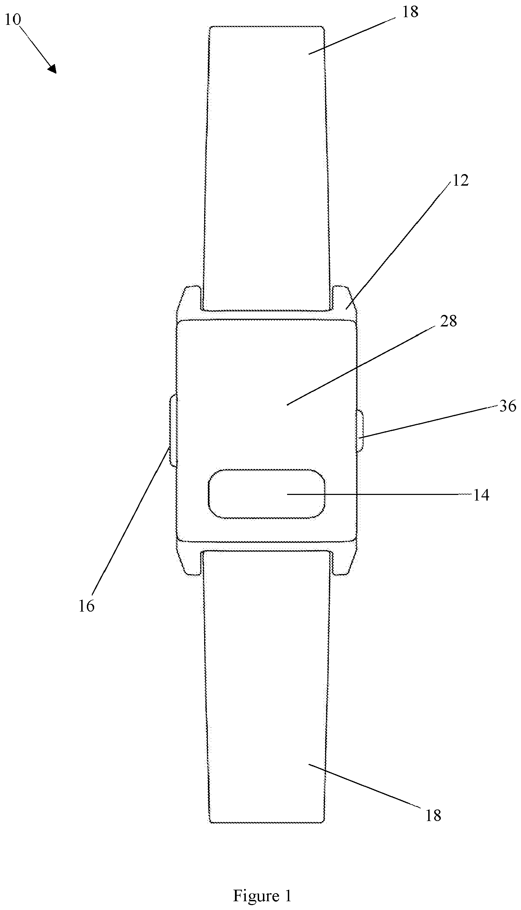

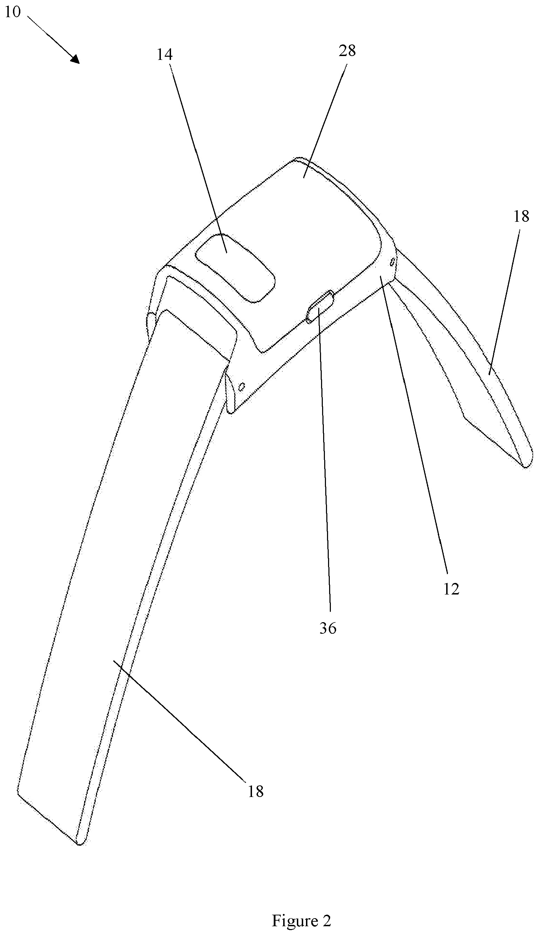

Referring firstly to Figures 1 to 3, a first embodiment of the wearable device for recording painkillers taken and a headache level is generally indicated at 10. The wearable device 10 comprises a body 12 with first input button 14 and second input button 16 disposed thereon, and a strap 18 attached to a first end and a second end of the body by a releasable connector (not shown).

The strap 18 is attached to a first end and a second end of the body 12 by a releasable connector (not shown). The strap 16 secures the wearable device to a user, preferably around a wrist, however, it may be sized and shaped so as to secure the wearable device to another location on the body.

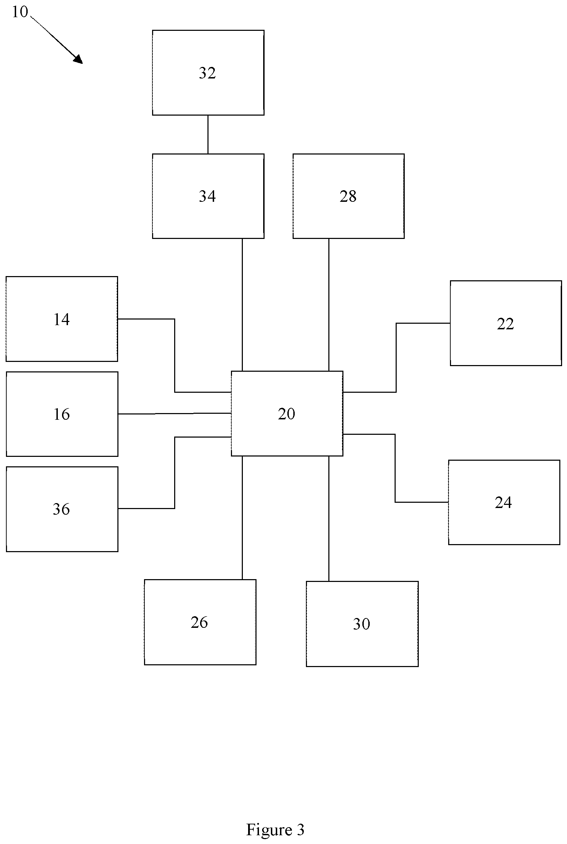

The body 12 has a front face, a rear face and side faces which enclose various components (as schematically illustrated in figure 3), such as a processor 20, a vibration motor 22, a buzzer 24, and a memory 26. A display 28 is disposed on the front face. In other embodiments, there is no display.

The first input button 14 is disposed on the front face and used to record a medicament event, such as the user taking a painkiller. The medicament dosing event is recorded by depressing the first input button 14 for a first predetermined length of time, preferably 3 seconds. After a delay, preferably 5 seconds, the processor 20 stores the medicament event in the memory 26. Subsequent operation of the first input button 14, within the delay, for a second predetermined length of time, preferably 3 seconds, removes the recorded medicament dosing event.

In the current embodiment, the processor 20 controls the vibration motor 22 to indicate that the first input button 14 has been operated for the required length of time, i.e. either the first or second predetermined lengths of time. The vibration motor will vibrate for a period of time which corresponds to the first or second predetermined lengths of time. In another embodiment, a first vibrational pattern may represent the medicament event being recorded and a second vibrational pattern may represent the medicament event being removed.

The second input button 16 is disposed on one of the side faces and is used to record the severity of a headache. To record the headache level, the user initially operates the second input button 16 to enter into an input mode in which subsequent operation of the second input button 16 allows for the headache level to be selected based on a predetermined scale. In the current embodiment, the predetermined scale sets 0 to represent no headache and 3 to represent the most severe.

The processor 20 controls the vibration motor 22 to produce vibrational feedback so as to indicate that the second input button 16 has been initially operated. This vibrational feedback also indicates the wearable device 10 has entered into the headache level input mode. Furthermore, the processor 20 controls the vibration motor 22 to indicate the headache level. However, there is a delay between the operations of the second input button 16 to set the headache level and the vibrational feedback indicating the headache level. This delay allows the user, in their debilitated state, to determine the selected headache level. During the input mode, if no input from the second input button 16 is detected in a first predetermined period, such as two seconds, the vibrational motor 22 produces vibrational feedback representing the headache level. For example, the headache level is set to 3 by operating the second input button 3 times then after 2 seconds of no operation the vibration motor produces two vibrational pulses.

A delay to the headache level being recorded is provided to allow a user to change the selected headache level. If no input from the second input button 16 is detected in a second predetermined period, such as 5 seconds, the wearable device 10 exits input mode and the headache level recorded is stored in the memory.

In the current embodiment, the wearable device 10 includes a wireless communications module 30 configured to send and receive wireless signals to and from the remote computer (not shown) using, for example, the Bluetooth (RTM) standard, or another comparable standard. In other embodiments, the communications module may also use a wired connection to send and receive data.

The wearable device 10 includes a battery 32 coupled to a charger 34 and a plurality of conductors (not shown) disposed on the rear face of the body 12. At least some of the plurality of conductors are configured to correspond and interface, in use, with a plurality of conductors disposed on a dock so as to charge the battery. In an alternative embodiment, the plurality of conductors may be disposed within a cable connector, such as a micro-USB connector. The plurality of conductors not used for charging may be coupled to the communication modules to allow for wired communication.

A screen selection button 36 is disposed on the side face opposite the second input button 16. The screen selection button 36 allows a user to select the screen to be displayed on display 28 without recording a medicament event or headache level. The default screen may present the user with information similar to a digital watch, such as a clock, a date and battery level.

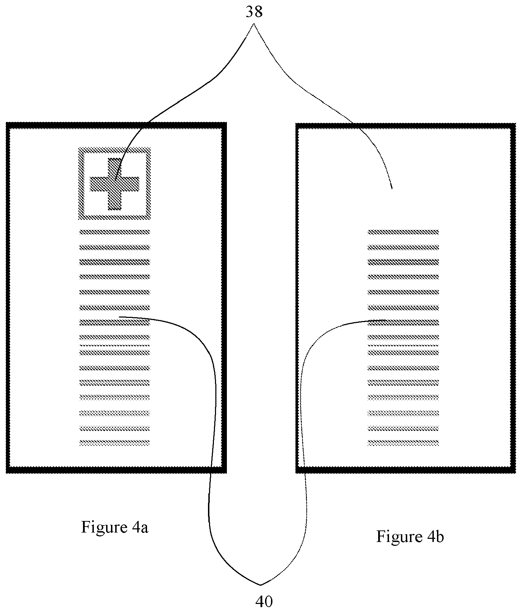

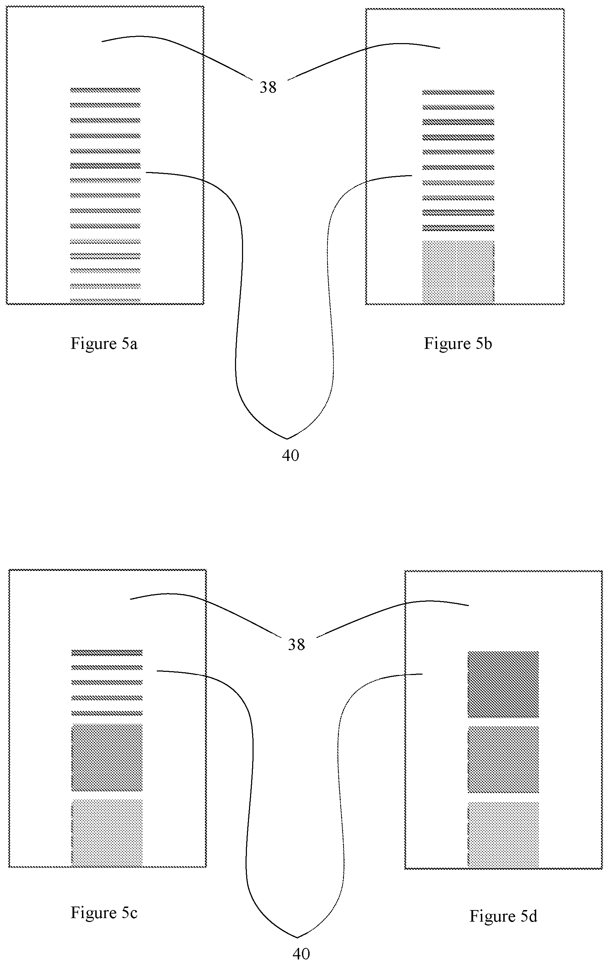

A painkiller/headache screen, as shown in figures 4a, 4b, 5a, 5b, 5c and 5d, is displayed to the user upon operation of either the first input button 14, the second input button 16, or the screen select button 30. The painkiller/headache screen includes a first display area 38 and a second display area 40. A painkiller symbol is displayed in the first display area 38 when a medicament event has been recorded during the present calendar day, as shown in figure 4a. A headache level indicator is displayed in the second display area 40. The indicator shows a visual representation of the headache level selected during the headache level input mode or a headache level stored on the same calendar day. For example, in the current embodiment, the second display area displays a scale formed from a series of parallel lines and the headache level is represented by solid blocks on the scale with the maximum number of blocks being equal to the predetermined scale, see figures 5a, 5b, 5c, and 5d.

The painkiller/headache screen also indicates the number of medicament events stored during the current calendar month. In the current embodiment, the processor 20 uses the data stored in the memory 26 to determine the number of painkillers taken in the current calendar month and changes the colour of the headache level indicator and the painkiller symbol. For example, when there have been no painkillers taken the headache level and painkiller symbol may be green, but when the number of painkillers taken is close to 15 the headache level indicator and painkiller symbol may be red.

In some embodiments, the wearable device 10 includes a pedometer to record the number of steps taken during a given day in the memory 26. A screen showing the number of steps taken during the day may be displayed on the display 28. The screen selection button 36 may be used to select the screen showing the number of steps taken.

The disclosed wearable device allows a user to record a headache at the time that it occurs and also record the taking of a painkiller, without reference to a screen. Separate buttons enable straightforward operation, when debilitated by a headache, and the feedback vibrations corresponding to the inputs enable a person to confirm that the correct inputs have been made. In the current embodiment an inclusion of a screen allows the user to ensure that the data has been correctly inputted after the headache has passed.

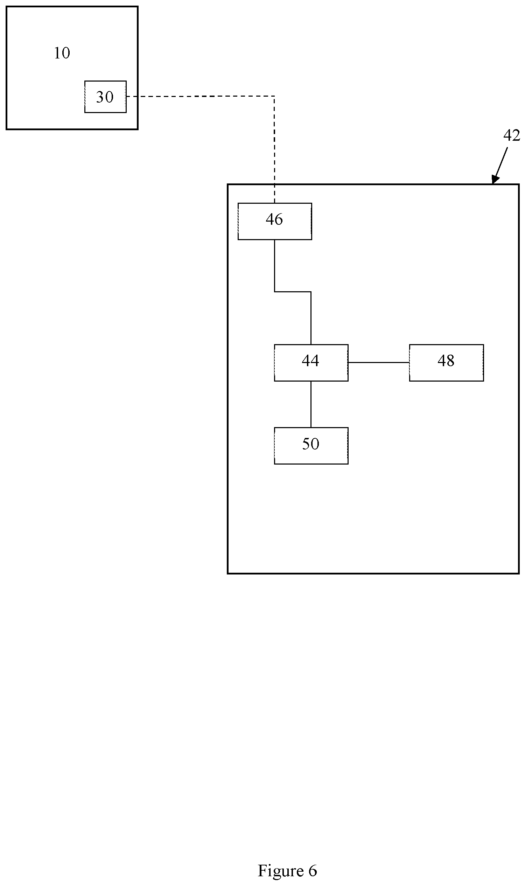

Figure 6 shows a remote device generally indicated at 42. The remote device comprises a communication module 44 for communicating with an external device such as the wearable device 10 or an intermediary device, a processor 46, a memory 48 and a display 50.

A remote device 42, such as a smart phone, tablet computer or other computing device, receives data transferred from the wearable device 10. The data may be transferred by a direct wired or wireless connection, or alternatively it may be transferred via an intermediary. For example, the data may be transferred to an application on a smart phone, or other similar device, and be stored on a remote cloud server to be accessed by the remote device. In other embodiments the remote device receives data from a plurality of wearable devices. The data received includes medicament dosing data, headache data, headache level data, time data, date data, and an ID associated with the wearable device. A memory within the remote device stores the received data. Analysis is performed on the stored data. Reports may be produced based on the received data. These reports may contain trends which allows a user of the remote device to analyse the data. An alert may be produced based on the analysis performed. For example, the remote device may produce an alert based on a user of a wearable device taking a painkiller for 15 or more days in a calendar month. The number of days and the number of months may vary, for example the remote device may produce an alert based on a user of the wearable device taking a painkiller for 10 or more days, preferably 11 days, in a calendar month or a three month period.

In some embodiments, it is only the user of the remote device which has access to the data. The remote device, or an app running on the remote device, may have security permissions which limit a user's ability to access or view the received data, analysed data and reports. For example, a physician, or other medical professional, may have access to the data received from the wearable device, while the user of the wearable device is blind to that data. This can be particularly useful to prevent patients from influencing the system consciously or unconsciously, particularly during trials.

The embodiments described above are provided by way of example only, and various changes and modifications will be apparent to persons skilled in the art without departing from the scope of the present invention as defined by the appended claims.

## Claims

1. A wearable device (10) for allowing an impaired user to record a medicament dosing event and a headache severity level during a headache, such as a migraine, without reference to a screen, the device comprising:
a first input button (14) and a second input button (16) disposed on separate faces of a body (12) of the wearable device so as to be identifiable to a user during the headache, the first and second input buttons being operable by being pressed and released; a vibration motor (22) housed within the body for producing vibrational feedback to the user during the headache; a processor (20) housed within the body; and a memory (26) housed within the body for storing data; wherein the processor is configured to:
record, within the memory (26), a medicament dosing event based on operation of the first input button (14), the medicament dosing event being binary;
record, within the memory (26), a headache severity level based on the number of operations of the second input button (16) within a predetermined time period, the headache severity level being a non-binary value; **characterised by**
the headache severity level being stored in the memory after the predetermined time period;
the vibration motor (22) being controlled to indicate the medicament dosing event based on the operation of the first input button (14); and
the vibration motor (22) being controlled, prior to the headache severity level being recorded within the memory, to indicate the headache severity level after a first delay following the number of operations of the second input button (16) for allowing the impaired user to determine or validate their input without reference to the screen and cancel an erroneous input.

2. A wearable device (10) as claimed in claim 1, in which the second input button (16) has to be initially operated to allow or prime the processor (20) to record the headache severity level, and the vibration motor (22) vibrates to indicate that the headache severity level can be set.

3. A wearable device (10) as claimed in any preceding claim, in which operation of the first input button (14) for at least a first predetermined length of time records the medicament dosing event after a second delay, and the vibration motor (22) vibrates for a period corresponding to the first predetermined length of time.

4. A wearable device (10) as claimed in claim 3, in which subsequent operation of the first input button (14) for at least a second predetermined length of time within the second delay removes the medicament dosing event, and the vibration motor (22) vibrates for a period corresponding to the second predetermined length of time.

5. A wearable device (10) as claimed in any preceding claim, in which there are at least two vibration patterns used to control the vibration motor (22), each vibration pattern includes at least one vibration pulse, wherein each vibration pattern identifies either operation of the first input button (14) or the second input button (16).

6. A wearable device (10) as claimed in any preceding claim, in which the wearable device has a display (28) for displaying information based on the stored data.

7. A wearable device (10) as claimed in claim 6, in which the wearable device further comprises a third input button (36) for controlling the display (28).

8. A wearable device (10) as claimed in any preceding claim, in which the processor (20) stores the date and time of the medicant dosing event and/or the headache event.

9. A wearable device (10) as claimed in any preceding claim, in which the wearable device has a communication module (30) for transmitting the stored data to a remote device for analysis and display.

10. A wearable device (10) as claimed in claim 9, in which the communication module (30) comprises at least one of a wireless communication module and a wired communication module.

11. A wearable device (10) as claimed in any preceding claim, in which the body (12) comprises a front face, a rear face, side faces, a first end and a second end.

12. A wearable device (10) as claimed in claim 11, in which the first input button (14) is disposed on the front face and the second input button (16) is disposed on one of the side faces.

13. A wearable device (10) as claimed in claim 11, in which the wearable device further comprises a strap (18) attached to the first and second ends of the body (12) by a releasable fastener.

14. A wearable device (10) as claimed in claim 13, in which the strap (18) comprises a further releasable fastener for securing the strap around the user.

15. A wearable device (10) as claimed in any preceding claim, further comprising a buzzer (24) or speaker for providing auditory feedback to the user, the processor configured to control the buzzer or speaker based on the operation of the first and second input buttons (14, 16).

## Patentansprüche

1. Tragbare Vorrichtung (10), die es einem beeinträchtigten Benutzer ermöglicht, ein Medikamentendosierungsereignis und eine Stufe des Kopfschmerzleidens während eines Kopfschmerzes, wie etwa einer Migräne, ohne Bezugnahme auf einen Bildschirm aufzuzeichnen, die Vorrichtung umfassend:
eine erste Eingabe-Schaltfläche (14) und eine zweite Eingabe-Schaltfläche (16), die auf getrennten Flächen eines Körpers (12) der tragbaren Vorrichtung angeordnet sind, um für einen Benutzer während des Kopfschmerzes identifizierbar zu sein, wobei die erste und die zweite Eingabe-Schaltfläche durch Drücken und Loslassen betätigt werden können; einen im Körper untergebrachten Vibrationsmotor (22) zur Erzeugung einer Vibrationsrückmeldung an den Benutzer während der Kopfschmerzen; einen im Körper untergebrachten Prozessor (20); und einen im Körper untergebrachten Speicher (26) zum Speichern von Daten; wobei der Prozessor so konfiguriert ist, dass er:
in dem Speicher (26) ein Medikamentendosierungsereignis basierend auf dem Vorgang der ersten Eingabe-Schaltfläche (14) aufzeichnet, wobei das Medikamentendosierungsereignis binär ist;
in dem Speicher (26) eine Stufe des Kopfschmerzleidens basierend auf der Anzahl der Vorgänge der zweiten Eingabe-Schaltfläche (16) innerhalb einer vorbestimmten Zeitperiode aufzeichnet, wobei die Stufe des Kopfschmerzleidens ein nicht-binärer Wert ist; **gekennzeichnet dadurch, dass**
die Stufe des Kopfschmerzleidens nach der vorbestimmten Zeitspanne in dem Speicher gespeichert wird;
der Vibrationsmotor (22) gesteuert wird, um das Medikamentendosierungsereignis basierend auf dem Vorgang der ersten Eingabe-Schaltfläche (14) anzuzeigen; und
der Vibrationsmotor (22) vor der Aufzeichnung des Kopfschmerzleidens im Speicher so gesteuert wird, dass er die Stufe des Kopfschmerzleidens nach einer ersten Verzögerung im Anschluss an die Anzahl der Vorgänge der zweiten Eingabe-Schaltfläche (16) anzeigt, um es dem beeinträchtigten Benutzer zu ermöglichen, seine Eingabe ohne Bezugnahme auf den Bildschirm zu bestimmen oder zu bestätigen und eine Fehleingabe zu löschen.

2. Tragbare Vorrichtung (10) nach Anspruch 1, wobei die zweite Eingabe-Schaltfläche (16) anfänglich betätigt werden muss, um dem Prozessor (20) die Aufzeichnung des Kopfschmerzleidens zu ermöglichen oder zu aktivieren, und der Vibrationsmotor (22) vibriert, um anzuzeigen, dass die Stufe des Kopfschmerzleidens eingestellt werden kann.

3. Tragbare Vorrichtung (10) nach einem der vorstehenden Ansprüche, wobei der Vorgang der ersten Eingabe-Schaltfläche (14) für mindestens eine erste vorbestimmte Zeitspanne das Medikamentendosierungsereignis nach einer zweiten Verzögerung aufzeichnet und der Vibrationsmotor (22) für eine Zeitspanne vibriert, die der ersten vorbestimmten Zeitspanne entspricht.

4. Tragbare Vorrichtung (10) nach Anspruch 3, wobei ein anschließender Vorgang der ersten Eingabe-Schaltfläche (14) für mindestens eine zweite vorbestimmte Zeitspanne innerhalb der zweiten Verzögerung das Medikamentendosierungsereignis aufhebt und der Vibrationsmotor (22) für einen Zeitraum vibriert, der der zweiten vorbestimmten Zeitspanne entspricht.

5. Tragbare Vorrichtung (10) nach einem der vorstehenden Ansprüche, wobei mindestens zwei Vibrationsmuster zur Steuerung des Vibrationsmotors (22) verwendet werden, wobei jedes Vibrationsmuster mindestens einen Vibrationsimpuls einschließt, wobei jedes Vibrationsmuster entweder den Vorgang der ersten Eingabe-Schaltfläche (14) oder der zweiten Eingabe-Schaltfläche (16) identifiziert.

6. Tragbare Vorrichtung (10) nach einem der vorstehenden Ansprüche, wobei die tragbare Vorrichtung eine Anzeige (28) zum Anzeigen von auf den gespeicherten Daten basierenden Informationen aufweist.

7. Tragbare Vorrichtung (10) nach Anspruch 6, wobei die tragbare Vorrichtung ferner eine dritte Eingabe-Schaltfläche (36) zur Steuerung der Anzeige (28) umfasst.

8. Tragbare Vorrichtung (10) nach einem der vorstehenden Ansprüche, wobei der Prozessor (20) das Datum und die Uhrzeit des Medikamentendosierungsereignisses und/oder des Kopfschmerzereignisses speichert.

9. Tragbare Vorrichtung (10) nach einem der vorstehenden Ansprüche, wobei die tragbare Vorrichtung ein Kommunikationsmodul (30) zum Übertragen der gespeicherten Daten an eine entfernte Vorrichtung zur Analyse und Anzeige aufweist.

10. Tragbare Vorrichtung (10) nach Anspruch 9, wobei das Kommunikationsmodul (30) mindestens eines von einem drahtlosen Kommunikationsmodul und einem drahtgebundenen Kommunikationsmodul umfasst.

11. Tragbare Vorrichtung (10) nach einem der vorstehenden Ansprüche, wobei der Körper (12) eine Vorderseite, eine Rückseite, Seitenflächen, ein erstes Ende und ein zweites Ende aufweist.

12. Tragbares Gerät (10) nach Anspruch 11, wobei die erste Eingabe-Schaltfläche (14) auf der Vorderseite und die zweite Eingabe-Schaltfläche (16) auf einer der Seitenflächen angeordnet ist.

13. Tragbare Vorrichtung (10) nach Anspruch 11, wobei die tragbare Vorrichtung ferner einen Riemen (18) umfasst, der durch einen lösbaren Verschluss am ersten und zweiten Ende des Körpers (12) befestigt ist.

14. Tragbare Vorrichtung (10) nach Anspruch 13, wobei das Riemen (18) einen weiteren lösbaren Verschluss zum Sichern des Bandes um den Benutzer herum umfasst.

15. Tragbare Vorrichtung (10) nach einem der vorstehenden Ansprüche, die ferner umfassen einen Buzzer (24) oder Lautsprecher, um dem Benutzer eine akustische Rückmeldung bereitzustellen, wobei der Prozessor so konfiguriert ist, dass er den Buzzer oder Lautsprecher basierend auf dem Vorgang der ersten und zweiten Eingabe-Schaltfläche (14, 16) steuert.

## Revendications

1. Dispositif à porter sur soi (10) pour permettre à un utilisateur dont les facultés sont affaiblies d'enregistrer un événement d'administration de médicament et un niveau d'intensité de céphalée lors d'une céphalée, par exemple une migraine, sans en référer à un écran, le dispositif comprenant :
un premier bouton d'entrée (14) et un deuxième bouton d'entrée (16) disposés sur des faces distinctes d'un corps (12) du dispositif à porter sur soi de manière à être identifiables par un utilisateur pendant la céphalée, les premier et deuxième boutons d'entrée pouvant être actionnés en étant pressés et relâchés ; un moteur de vibration (22) logé dans le corps pour produire un retour vibratoire à l'utilisateur pendant la céphalée ; un processeur (20) logé dans le corps ; et une mémoire (26) logée dans le corps pour stocker des données ; dans lequel le processeur est configuré pour :
enregistrer, dans la mémoire (26) un événement d'administration de médicament sur la base d'un actionnement du premier bouton d'entrée (14), l'événement d'administration de médicament étant binaire ;
enregistrer, dans la mémoire (26), un niveau d'intensité de céphalée sur la base du nombre d'actionnements du deuxième bouton d'entrée (16) au cours d'une période de temps prédéterminée, le niveau d'intensité de céphalée étant une valeur non binaire ; **caractérisé par** le niveau d'intensité de céphalée étant stocké dans la mémoire après la période de temps prédéterminée ;
le moteur de vibration (22) étant commandé pour indiquer l'événement d'administration de médicament sur la base de l'actionnement du premier bouton d'entrée (14) ; et
le moteur de vibration (22) étant commandé, avant que le niveau d'intensité de céphalée ne soit enregistré dans la mémoire, pour indiquer le niveau d'intensité de céphalée après un premier retard suivant le nombre d'actionnements du deuxième bouton d'entrée (16) pour permettre à l'utilisateur dont les facultés sont affaiblies de déterminer ou valider son entrée sans en référer à l'écran et annuler une entrée erronée.

2. Dispositif à porter sur soi (10) selon la revendication 1, dans lequel le deuxième bouton d'entrée (16) doit d'abord être actionné pour autoriser ou préparer le processeur (20) à enregistrer le niveau d'intensité de céphalée, et le moteur de vibration (22) vibre pour indiquer que le niveau d'intensité de céphalée peut être défini.

3. Dispositif à porter sur soi (10) selon l'une quelconque des revendications précédentes, dans lequel l'actionnement du premier bouton d'entrée (14) pendant au moins une première durée prédéterminée enregistre l'événement d'administration de médicament après un deuxième retard, et le moteur de vibration (22) vibre pendant une période correspondant à la première durée prédéterminée.

4. Dispositif à porter sur soi (10) selon la revendication 3, dans lequel un actionnement ultérieur du premier bouton d'entrée (14) pendant au moins une deuxième durée prédéterminée au cours du deuxième retard supprime l'événement d'administration de médicament, et le moteur de vibration (22) vibre pendant une période correspondant à la deuxième durée prédéterminée.

5. Dispositif à porter sur soi (10) selon l'une quelconque des revendications précédentes, dans lequel il existe au moins deux modèles de vibration utilisés pour commander le moteur de vibration (22), chaque modèle de vibration inclut au moins une impulsion vibratoire, dans lequel chacun modèle de vibration identifie soit l'actionnement du premier bouton d'entrée (14), soit du deuxième bouton d'entrée (16).

6. Dispositif à porter sur soi (10) selon l'une quelconque des revendications précédentes, dans lequel le dispositif à porter sur soi présente un dispositif d'affichage (28) pour afficher des informations sur la base des données stockées.

7. Dispositif à porter sur soi (10) selon la revendication 6, dans lequel le dispositif à porter sur soi comprend en outre un troisième bouton d'entrée (36) pour commander le dispositif d'affichage (28).

8. Dispositif à porter sur soi (10) selon l'une quelconque des revendications précédentes, dans lequel le processeur (20) stocke la date et l'heure de l'événement d'administration de médicament et/ou de l'événement de céphalée.

9. Dispositif à porter sur soi (10) selon l'une quelconque des revendications précédentes, dans lequel le dispositif à porter sur soi présente un module de communication (30) pour transmettre les données stockées à un dispositif distant en vue de l'analyse et de l'affichage.

10. Dispositif à porter sur soi (10) selon la revendication 9, dans lequel le module de communication (30) comprend au moins l'un parmi un module de communication sans fil et un module de communication filaire.

11. Dispositif à porter sur soi (10) selon l'une quelconque des revendications précédentes, dans lequel le corps (12) comprend une face avant, une face arrière, des faces latérales, une première extrémité et une deuxième extrémité.

12. Dispositif à porter sur soi (10) selon la revendication 11, dans lequel le premier bouton d'entrée (14) est disposé sur la face avant et le deuxième bouton d'entrée (16) est disposé sur l'une des faces latérales.

13. Dispositif à porter sur soi (10) selon la revendication 11, dans lequel le dispositif à porter sur soi comprend en outre une bande (18) fixé aux première et deuxième extrémités du corps (12) par une attache libérable.

14. Dispositif à porter sur soi (10) selon la revendication 13, dans lequel la bande (18) comprend une autre attache libérable pour fixer la bande autour de l'utilisateur.

15. Dispositif à porter sur soi (10) selon l'une quelconque des revendications précédentes, comprenant en outre un avertisseur sonore (24) ou un haut-parleur pour fournir un retour sonore à l'utilisateur, le processeur étant configuré pour commander l'avertisseur sonore ou le haut-parleur sur la base de l'actionnement des premier et deuxième boutons d'entrée (14, 16).
